Europäisches Patentamt

**European Patent Office**

**Office européen des brevets**

(11) Numéro de publication: **0 273 822**
**A2**

(12) **DEMANDE DE BREVET EUROPEEN**

(21) Numéro de dépôt: **87402889.7**

(22) Date de dépôt: **17.12.87**

(51) Int. Cl.⁴: **A 61 K 31/195**
**A 61 K 31/60**

(30) Priorité: **22.12.86 FR 8617991**

(43) Date de publication de la demande:
**06.07.88 Bulletin 88/27**

(84) Etats contractants désignés:
**AT BE CH DE FR GB IT LI LU NL SE**

(71) Demandeur: **SYNTHELABO**
**58 rue de la Glacière**
**F-75013 Paris (FR)**

(72) Inventeur: **Benjamin, Lennette M.D. Rockfeller Institute**
**New York The Rockfeller University**
**1230 York Avenue New York, 10021-6399 (US)**

(74) Mandataire: **Thouret-Lemaitre, Elisabeth et al**
**Service Brevets - SYNTHELABO 58, rue de la Glacière**
**F-75013 Paris (FR)**

(54) Compositions pharmaceutiques contenant de l'acétylsalicylate de lysine.

(57) Composition pharmaceutique utile pour le traitement de la drepanocytose caractérisée en ce qu'elle contient de l'acétyl-salicylate de lysine.

## Description

### COMPOSITIONS PHARMACEUTIQUES CONTENANT DE L'ACETYLSALICYLATE DE LYSINE

La présente invention a pour objet des compositions pharmaceutiques contenant de l'acétylsalicylate de lysine.

La Demanderesse poursuivant sa recherche relative à l'acétylsalicylate de lysine a constaté que l'acétylsalicylate de lysine, administré par voie intraveineuse, est très actif lors du traitement de malades atteints de la drépanocytose. Ces derniers souffrent de crises de douleurs difficiles à traiter. Plusieurs médicaments se sont en effet révélés inactifs ou insuffisamment actifs pour traiter ces douleurs. L'acétylsalicylate de lysine par ses propriétés analgésiques et antiinflammatoires permet un traitement efficace des malades.

Il est en effet constaté qu'après administration par voie intraveineuse de plusieurs doses (en moyenne 4 à 5 doses) d'acétylsalicylate de lysine, chaque dose étant équivalente à 500 mg d'acide acétylsalicylique, les douleurs des patients ont soit disparu soit diminué en grande partie.

La durée du traitement est variable selon les malades et l'intensité des douleurs. On peut constater en effet chez certains malades dés l'administration intraveineuse de la troisième ou de la quatrième dose d'acétylsalicylate de lysine, une diminution de l'intensité de la douleur, une réduction concomitante des gonflements et l'abolition du "syndrome de douleur de la poitrine".

Les compositions pharmaceutiques de l'invention contiennent l'acétylsalicylate de lysine en quantité correspondant à 500 mg ou 1000 mg d'acide acétylsalicylique en association avec tout excipient approprié, en particulier tout excipient approprié par la voie intraveineuse.

Un exemple de composition pharmaceutique est le suivant :

acétylsalicylate de lysine    0,9 g    1,8 g
glycine    0,1 g    0,2 g

que l'on dissout extamporanément dans 5 ml d'eau pour préparations injectables.

pour la fabrication de moyens destinés à traiter la drépanocytose.

### Revendications

1. Composition pharmaceutique utile pour le traitement de la drepanocytose caractérisée en ce qu'elle contient de l'acétylsalicylate de lysine.

2. Composition pharmaceutique utile pour le traitement de la drepanocytose caractérisée en ce qu'elle contient de l'acétylsalicylate de lysine en association avec tout excipient approprié.

3. Composition pharmaceutique utile pour le traitement de la drepanocytose caractérisée en ce qu'elle contient de l'acétylsalicylate de lysine en association avec tout excipient approprié pour l'administration par voie intraveineuse.

4. Utilisation de l'acétylsalicylate de lysine